# DEMANDE DE BREVET EUROPEEN

(11) **EP 1 911 520 A1**
(43) Date de publication de la demande: **16.04.2008**
(21) Numéro de dépôt: 06405432.3
(22) Date de dépôt: 10.10.2006
(51) Int. Cl.: B04B 5/04, B04B 11/02, B04B 11/08

(54) **Ensemble jetable pour la séparation de sang ou le lavage de composant du sang**

(71) Demandeur: Rochat, Jean-Denis, 1272 Genolier (CH)
(72) Inventeur: Rochat, Jean-Denis, 1272 Genolier (CH)
(74) Mandataire: Savoye, Jean-Paul

(57) **Abrégé**

Une enceinte de centrifugation (1) en plastique rigide, comporte un élément (1b) conformé pour venir en prise avec un organe d'entraînement d'une machine de centrifugation (M) et un organe de mise en communication (5) de l'enceinte (1) avec l'extérieur en plastique rigide, traversé par un conduit (5a) pour alimenter cette enceinte (1) et par deux conduits (5b, 5c) pour évacuer les constituants séparés. Un support (3) en plastique rigide dans lequel est formé un réseau de canaux (4a, 4b, 4c) sert à relier les conduits (5a, 5b, 5c) respectivement à un réservoirs d'alimentation (P1) et de réception (P2, P3), des éléments de fermeture latérale étanche (6) de ce réseau de canaux (4a, 4b, 4c). Les extrémités des conduits (5a, 5b, 5c) sont adjacentes aux extrémités respectives des canaux (4a, 4b, 4c). Les extrémités des conduits (5a, 5b, 5c) ont des surfaces complémentaires aux surfaces d'extrémités des canaux respectifs (4a, 4b, 4c) et ces surfaces complémentaires sont fixées jointivement l'une à l'autre.

## Description

La présente invention se rapporte à un ensemble jetable pour la séparation de sang ou le lavage de composant du sang par centrifugation, comprenant une enceinte de centrifugation circulaire en un matériau plastique rigide, comportant, à une extrémité située sur son axe de rotation, un élément conformé pour venir en prise avec un organe d'entraînement d'une machine de centrifugation et à une extrémité opposée un organe de mise en communication de ladite enceinte avec l'extérieur en un matériau plastique rigide, relié à ladite enceinte de centrifugation par un joint tournant et traversé par un conduit pour alimenter cette enceinte et par au moins conduit de sortie pour évacuer un des constituants séparés, un support en un matériau plastique rigide dans lequel est formé un réseau de canaux pour relier lesdits conduits respectivement à un réservoir d'alimentation pour le sang à séparer et à des réservoirs de réception respectifs des constituants séparés, des éléments de fermeture latérale étanche de ce réseau de canaux et des moyens pour faire circuler les liquides à travers ce réseau de canaux, du réservoir d'alimentation aux réservoirs de réception.

Les ensembles de centrifugation à usage unique utilisés pour la séparation des composants sanguins à partir du sang complet comportent deux parties principales: une partie mobile correspondant à la chambre de centrifugation et une partie fixe comprenant les poches de stockage des composants sanguins, des tubulures souples, ainsi que toutes les parties destinées à être connectées à la machine pilotant le processus de séparation des constituants sanguins, comme les interfaces avec les capteurs de pression, les pompes, les détecteurs d'air, les clamps, notamment. La chambre de centrifugation peut être réalisée en matière plastique rigide.

De façon générale, le terme rigide utilisé dans la description et les revendications pour qualifier les matériaux plastiques utilisés se rapporte à des matériaux qui ne sont ni flexibles, ni mous, ni souples, c'est-à-dire à des matériaux plastiques susceptibles de conserver leur forme initiale dans les conditions d'utilisation pour lesquelles ils ont été conçus.

Dans les ensembles de centrifugation susmentionnés, la connexion entre la partie mobile et la partie fixe est toujours réalisée par des tubulures souples qui gênent considérablement le montage de l'ensemble de centrifugation sur la machine, augmentant sensiblement le temps de travail de l'opérateur, ainsi que le risque d'un montage défectueux.

On a déjà proposé d'intégrer divers éléments d'un dispositif de séparation du sang sur un support rigide, sans pour autant arriver à supprimer les conduits souples entre la chambre de centrifugation et la partie fixe de l'ensemble.

Le WO 8402473 montre une structure moulée pour une machine de fractionnement du plasma comprenant des canaux d'écoulement de fluide et un filtre à membrane pour séparer le plasma du sang complet. Il ne s'agit donc pas de séparation par centrifugation.

Le US 20040245189 se rapporte à un ensemble de séparation à usage unique comprenant une cassette comportant un bâti en plastique moulé par injection qui supporte une tubulure soudée au bâti par ultra-sons et une chambre de centrifugation à écoulement continu. La chambre de centrifugation est reliée de manière amovible au bâti de la cassette pour pouvoir être insérée facilement dans une cuvette d'entraînement de la centrifugeuse pendant l'installation de la cassette, en sorte que la chambre de centrifugation est découplée du bâti de la cassette lors de la fermeture de la porte de l'appareil de centrifugation. La liaison entre les conduits de liaison fixes pour relier la chambre de centrifugation avec l'extérieur et les conduits respectifs solidaires de la chambre de centrifugation est réalisée par une pièce cylindrique tournante présentant sur sa face externe une série de canaux annulaires, une pièce fixe présentant une face adjacente à la face externe de la pièce cylindrique tournante est traversée par des conduits débouchant dans cette face adjacente, à des distances radiales respectives de l'axe de la pièce cylindrique tournante choisies pour mettre chaque conduit en communication avec un canal annulaire de la pièce tournante. L'étanchéité entre la pièce cylindrique tournante et la pièce fixe est assurée par une pression élastique des faces adjacentes de ces deux pièces. Etant donné la vitesse de rotation de la chambre de centrifugation, une telle solution pose de sérieux problèmes d'échauffement qui est susceptible de dégrader le sang traité passant dans les canaux annulaires ménagés sur la pièce cylindrique tournante, ainsi que dans les conduits de la pièce fixe pressée de manière élastique contre la pièce tournante, tournant à plusieurs milliers de tours/min pour garantir l'étanchéité de la circulation des liquides.

Le but de la présente invention est de remédier au moins en partie à ces inconvénients.

A cet effet, la présente invention a pour objet un ensemble jetable pour la séparation de sang par centrifugation du type susmentionné, selon la revendication 1.

Les dessins annexés illustrent, schématiquement et à titre d'exemple, deux formes d'exécution de l'ensemble jetable pour la séparation de sang, objet de la présente invention.
La figure 1 est une vue en perspective d'une machine de centrifugation sur laquelle est monté l'ensemble jetable;
la figure 2 est une vue en perspective de l'ensemble jetable seul;

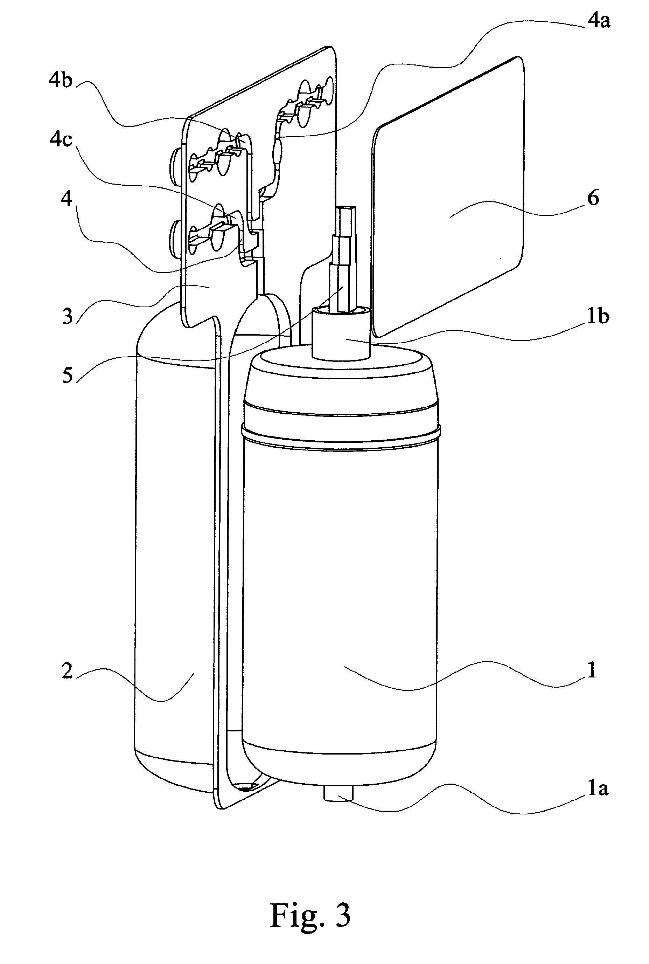

la figure 3 est une vue en perspective éclatée de l'ensemble jetable de la figure 2;
la figure 4 est une vue en coupe selon IV-IV de la figure 2;
la figure 5 est une vue en coupe selon V-V de la figure 6;
la figure 6 est une vue en coupe selon VI-VI de la figure 4;
la figure 7 est une vue de détail en coupe selon VII-VII de la figure 1;
la figure 8 est une vue en élévation latérale, partiellement en coupe, d'une seconde forme d'exécution de l'invention;
la figure 9 est une variante de la figure 4.

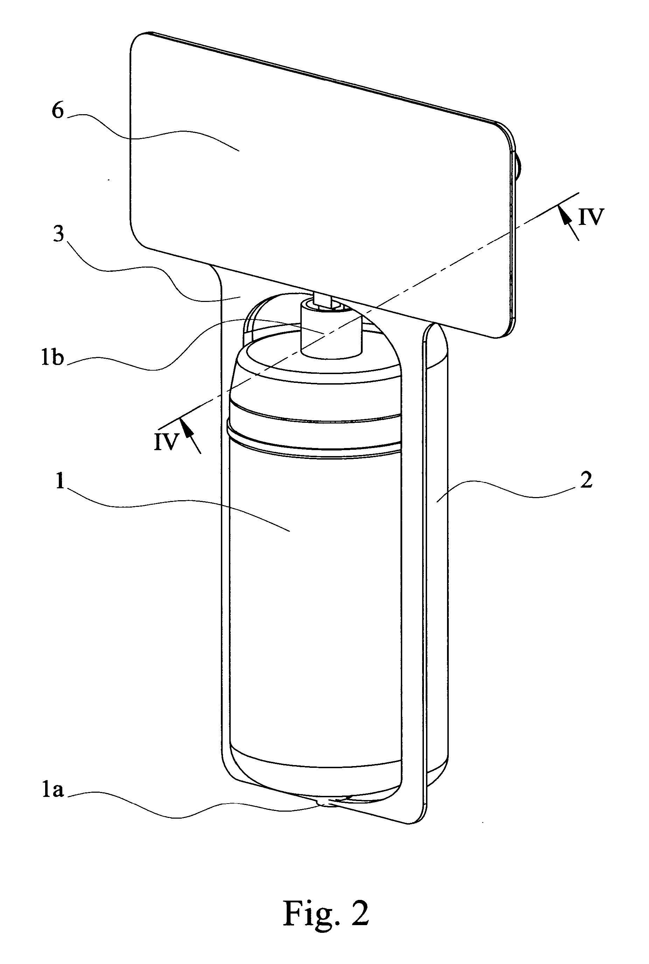

L'ensemble jetable pour la séparation de sang ou le lavage de composant du sang par centrifugation est illustré par la figure 2. Il comporte une chambre ou enceinte de centrifugation 1 présentant la forme d'un cylindre allongé logé en partie dans un carter semi-cylindrique 2 solidaire d'un support 3 dans lequel est formé un réseau de canaux 4. De préférence, ce support 3 est un support généralement plan, seul le réseau de canaux 4 formant un relief par rapport à ce plan. Tous ces éléments sont réalisés en un matériau plastique rigide, de préférence par injection. Les canaux 4 sont ouverts latéralement sur une face du support plan 3, une membrane souple 6 étant fixée par soudage ou par collage sur la face du support plan sur laquelle s'ouvrent les canaux 4, en sorte que ces canaux 4 forment alors des conduits ouverts seulement à leurs deux extrémités. Cette membrane 6 est réalisée en plastique souple tel que silicone, PUR, PVC plus plastifiant, EPDM.

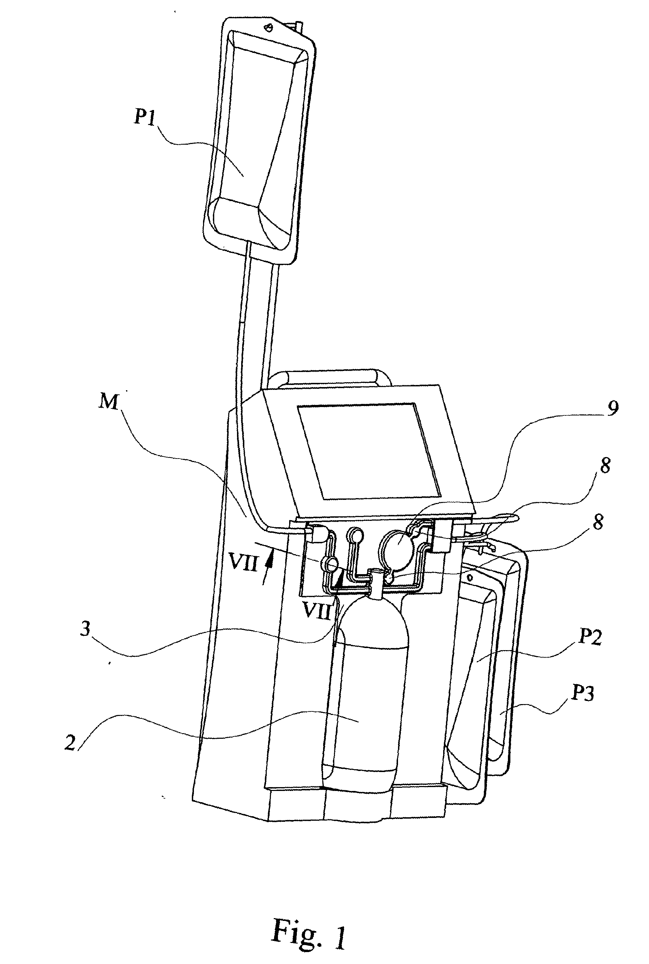

L'enceinte de centrifugation 1 comporte un élément d'accouplement 1a coaxial à son axe de rotation et faisant saillie à son extrémité inférieure, à travers une ouverture ménagée dans le fond du carter semi-cylindrique 2. Cet élément d'accouplement 1a présente intérieurement un logement de forme non circulaire pour venir en prise avec un organe d'entraînement (non représenté) de forme complémentaire, de la machine de centrifugation M, illustrée sur la figure 1. L'accouplement de l'enceinte de centrifugation avec l'organe d'entraînement de la machine de centrifugation correspond à celui d'une tête de vis, de préférence un type de tête de vis destiné à venir en prise avec un tourne-vis électrique, dont il existe une grande variété. D'autres systèmes d'entraînement sont également envisageables. L'extrémité opposée de l'enceinte de centrifugation 1 présente une ouverture tubulaire 1b, concentrique à l'axe de rotation de l'enceinte, pour recevoir un organe 5 de mise en communication de cette enceinte 1 avec l'extérieur. Un joint tournant est prévu au niveau de l'ouverture tubulaire 1b. Ce joint peut être matériel ou dynamique et sert à isoler l'enceinte de centrifugation de toute contamination extérieure. Par joint dynamique, on entend tout dispositif sans joint matériel apte à former une barrière stérile s'opposant à l'entrée d'air dans la chambre de centrifugation, par exemple en créant une légère surpression dans la chambre, engendrant ainsi une fuite de gaz sortant de cette chambre, empêchant de ce fait l'entrée d'air extérieur.

L'organe 5 de mise en communication de l'enceinte de centrifugation 1 avec l'extérieur comporte une partie interne pour alimenter l'enceinte 1 en sang à centrifuger et pour en évacuer les composants séparés. Etant donné que le mode de séparation utilisé par l'enceinte de centrifugation 1 ne fait pas partie de l'invention et n'est pas nécessaire à sa compréhension, il suffit de spécifier que l'organe 5 de mise en communication comporte un conduit central 5a pour amener du sang dans l'enceinte et au moins deux conduits périphériques 5b, 5c pour évacuer les composants séparés, notamment le plasma et les érythrocytes.

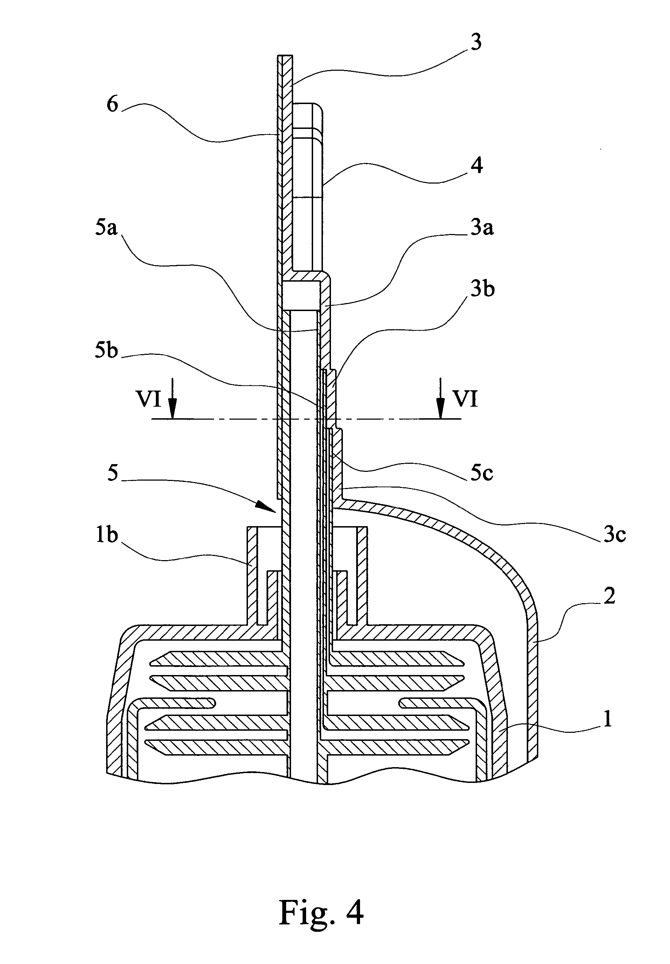

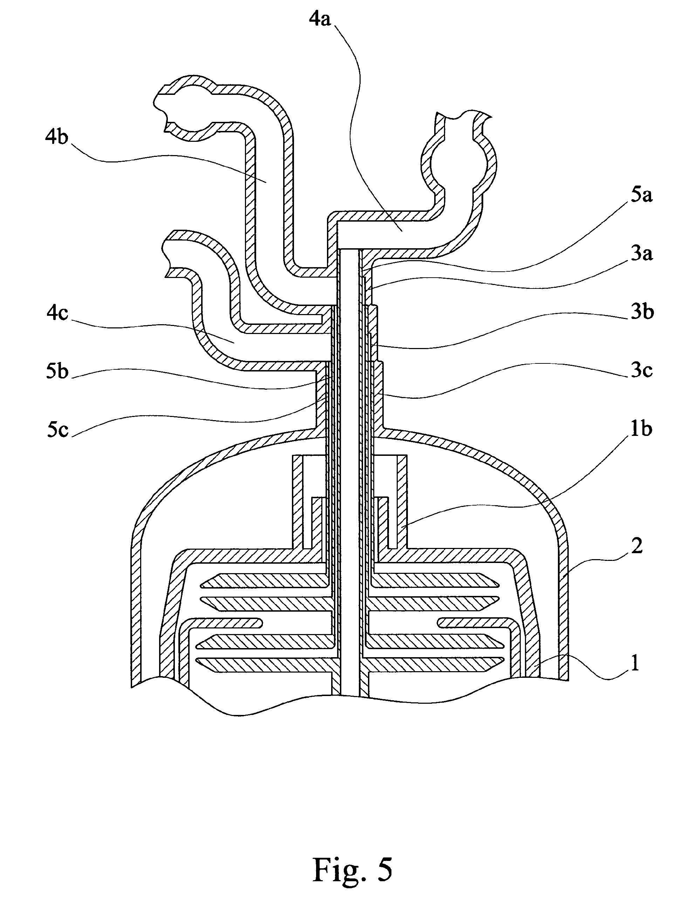

Comme on peut le voir sur les figures 4 et 5, les extrémités externes des conduits 5a, 5b, 5c sont étagées et se logent dans trois sièges respectifs 3a, 3b, 3c, de formes complémentaires, ménagés dans le support plan 3 et contre lesquels les surfaces adjacentes respectives des conduits 5a, 5b, 5c et des sièges 3a, 3b, 3c sont fixées les unes aux autres, de préférence, par soudage par ultra-sons. Chacun des sièges 3a, 3b, 3c fait communiquer les conduits 5a, 5b, 5c avec un des canaux 4a, 4b, 4c du réseau de canaux 4 ménagés dans le support plan 3.

Grâce au fait qu'aussi bien le carter 2 et le support plan 3, qui forment avantageusement une seule pièce obtenue par injection, que l'organe 5 de mise en communication de l'enceinte de centrifugation sont réalisés en matière plastique dur, lorsque cet ensemble de centrifugation est assemblé suite à la fixation des extrémités externes des conduits 5a, 5b, 5c de l'organe 5 dans les sièges respectifs 3a, 3b, 3c du support plan 3, cet ensemble de centrifugation forme un tout non déformable qui peut être monté de ce fait très facilement d'une seule main sur la machine de centrifugation M. Les extrémités ouvertes des conduits, formés par les canaux 4a, 4b, 4c fermés latéralement par la membrane 6, sont préalablement reliées à des poches souples P1, P2, P3, contenant respectivement le sang et au moins le plasma et les érythrocytes, et ne nécessitent donc aucun montage.

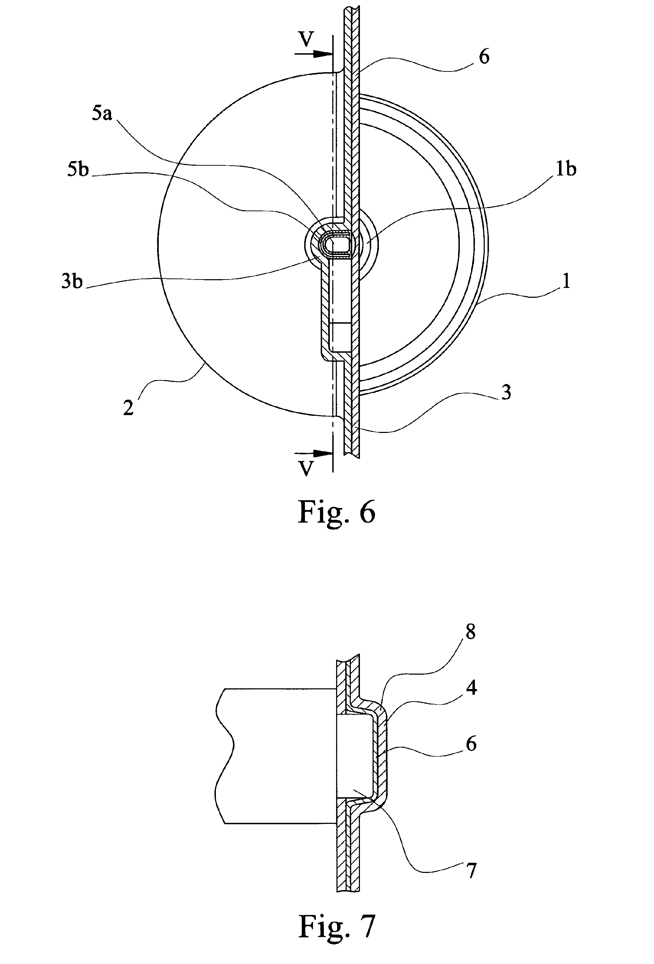

La membrane souple 6 sert à fermer latéralement les canaux 4a, 4b, 4c et en même temps, comme illustré par la figure 7, elle permet à des vérins 7 de la machine de centrifugation de déformer cette membrane 6 à des endroits déterminés. Ces vérins 7 permettent de commander l'ouverture et la fermeture des canaux 4a, 4b, 4c, servant ainsi de clamps pour contrôler l'écoulement dans les différents conduits en déformant la membrane 6 dans une cavité 8 formée le long des canaux 4a, 4b, 4c. Un autre vérin permet aussi de commander des opérations de pompage pour faire circuler le liquide dans les canaux 4a, 4b, 4c en déformant la membrane 6 dans une cavité plus grande 9, telle que celle illustrée à la figure 1. A cet effet ce vérin de pompage est déplacé en synchronisation avec les vérins 7 servant de clamps.

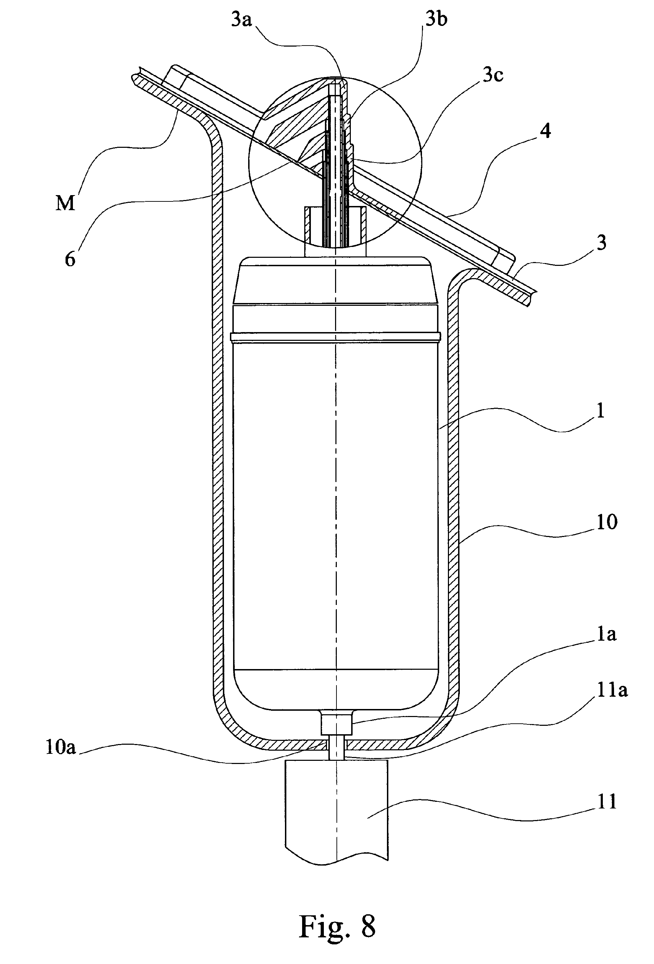

La seconde forme d'exécution illustrée par la figure 8 diffère de la forme d'exécution précédente essentiellement dans le fait que le carter 2 est supprimé. Le support plan 3 dans lequel est formé le réseau de canaux 4 est réalisé par injection de matière plastique rigide dans un moule. On retrouve dans cette forme d'exécution, comme dans la forme d'exécution précédente, les trois sièges 3a, 3b, 3c dans lesquels sont fixés, de préférence, par soudage par ultra-sons, les conduits respectifs 5a, 5b, 5c pour les mettre en communication avec les canaux respectifs du réseau de canaux 4 ménagé dans le support 3. Au lieu que le plan du support plan 3 soit parallèle à l'axe de rotation de l'enceinte de centrifugation 1, il est incliné par rapport à cet axe de rotation. L'angle d'inclinaison est donné par l'inclinaison de la surface supérieure de la machine de centrifugation. On pourrait aussi imaginer que cette surface supérieure est horizontale et que le support 3 est alors perpendiculaire à l'axe de rotation de la cuvette de centrifugation 1.

Dans cette variante, le carter 2 qui était solidaire du support 3 est remplacé par un logement 10 de la machine de centrifugation M, dont le fond présente une ouverture 10a pour permettre le passage de l'arbre d'entraînement 11a du moteur d'entraînement 11 dont l'extrémité vient en prise avec l'élément d'accouplement la de l'enceinte de centrifugation 1. Comme dans la forme d'exécution précédente, les canaux 4 sont fermés latéralement par une membrane souple 6. On peut aussi imaginer de remplacer le carter 2 de la première forme d'exécution par une paroi de la machine M.

Cette variante sans le carter 2 de la forme d'exécution précédente permet de réduire le prix de la partie jetable. Le logement 10 de la machine de centrifugation M dans lequel l'enceinte de centrifugation est logée permet aussi de donner une meilleure sécurité que le carter 2 de l'ensemble jetable de la forme d'exécution précédente.

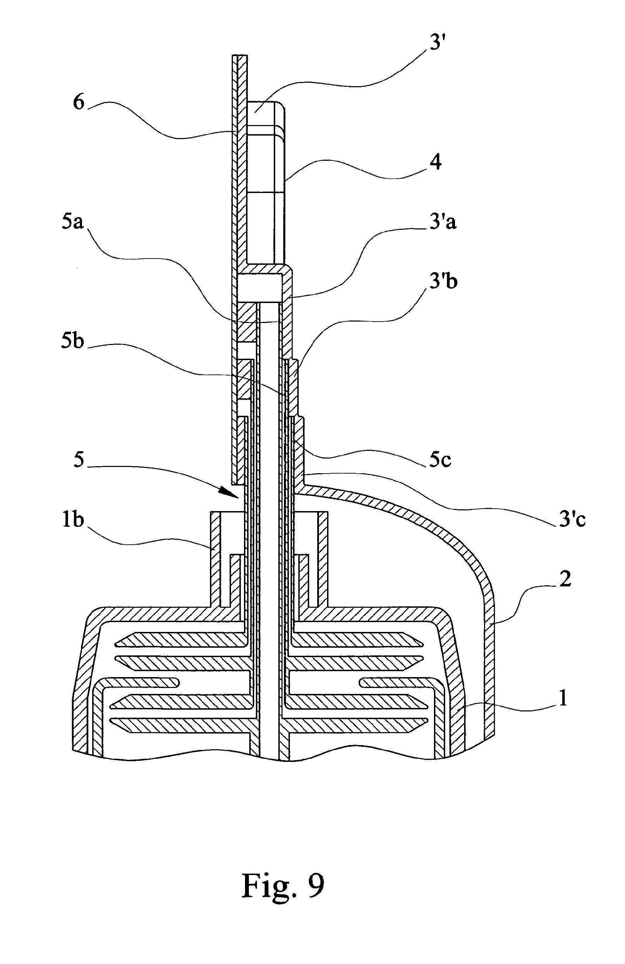

La figure 9 illustre une variante dans laquelle les sièges 3'a, 3'b et 3'c du support 3' pour la fixation des conduits 5a, 5b, 5c, ne sont plus semi-cylindriques, comme dans l'exemple de la figure 4, mais sont formés par des parties cylindriques qui entourent de ce fait complètement les extrémités des conduits 5a, 5b, 5c permettant de réaliser une meilleure fixation.

Par contre, alors que le support 3 et le carter 2 de la figure 4 peuvent être réalisés par déformation à chaud d'une feuille de matière thermoplastique dans l'empreinte d'un moule, la variante de la figure 9 ne peut être obtenue que par une technique d'injection, donc plus chère.

Les exemples décrits précédemment se rapportent à l'utilisation de l'ensemble jetable pour la séparation du sang, notamment la séparation en continu de sang. Ce même ensemble pourrait tout aussi bien être utilisé pour le lavage de produits sanguins par centrifugation.

Il peut s'agir par exemple de la déglycérolisation de concentré de globules rouges avant leur injection à un patient, le glycérol étant un composé ajouté au concentré globulaire pour améliorer sa conservation, sans hémolyse, à basse température.

Il peut également s'agir de lavage du sang destiné à l'autotransfusion. Dans ce cas, le sang du patient est collecté par des drains ou par une canule d'aspiration. Après avoir été filtré grossièrement, les globules rouges sont concentrés par centrifugation, puis lavés par injection de solution saline dans la chambre de centrifugation, pour emporter les impuretés et les séparer du concentré de globules rouges, plus dense. Les globules rouges lavés sont finalement extraits et collectés dans une poche de collection, avant leur ré-injection au patient.

On peut encore imaginer de laver le sang en discontinu. A cet effet, le sang à traiter est injecté dans la chambre de centrifugation via le conduit central 5a. Dans ce cas, l'ensemble jetable ne comporte alors qu'un seul conduit de sortie 5b par lequel la fraction la moins dense du liquide est extraite. Ensuite, l'enceinte de centrifugation 1 est arrêtée pour permettre à la fraction résiduelle plus dense de tomber au fond de cette enceinte. Cette fraction résiduelle est alors extraite par siphonnage par le conduit d'alimentation 5a.

Les systèmes de séparation sanguine ou d'autotransfusion actuels utilisent des pompes pour contrôler les débits entrants et sortants de l'enceinte de centrifugation. Il peut s'agir de pompes péristaltiques, nécessitant la présence de tuyaux souples sur l'ensemble à usage unique, ou de pompes à membranes dans le cas d'un ensemble semi-rigide, tel que décrit dans les exemples précédents.

Il est cependant également possible de faire circuler le liquide vers l'enceinte de centrifugation 1 en surélevant la poche d'alimentation P1 par rapport à l'enceinte de centrifugation, de manière à créer une colonne de liquide motrice, tel que montré sur la figure 1, et en ajustant la valeur du débit au moyen d'un étranglement réglable à l'aide d'un vérin 7, comme illustré par la figure 7.

De même, la pression nécessaire au transfert des composants sortant de l'enceinte de centrifugation jusqu'aux poches de collection P2, P3 peut être obtenue par transformation de l'énergie cinétique de rotation des liquides à l'intérieur de l'enceinte de centrifugation, en énergie potentielle de pression lors de leur extraction. L'ajustement du débit est alors également obtenu au moyen d'un étranglement réglable.

L'utilisation de pompes n'est donc pas indispensable.

## Revendications

1. Ensemble jetable pour la séparation de sang ou le lavage de composant du sang par centrifugation, comprenant une enceinte de centrifugation circulaire (1) en un matériau plastique rigide, comportant, à une extrémité située sur son axe de rotation, un élément (1b) conformé pour venir en prise avec un organe d'entraînement d'une machine de centrifugation (M) et à une extrémité opposée un organe de mise en communication (5) de ladite enceinte (1) avec l'extérieur en un matériau plastique rigide, relié à ladite enceinte de centrifugation par un joint tournant et traversé par un conduit (5a) pour alimenter cette enceinte (1) et par au moins un conduit de sortie (5b, 5c) pour évacuer au moins un des constituants séparés, un support (3) en un matériau plastique rigide dans lequel est formé un réseau de canaux (4a, 4b, 4c) pour relier lesdits conduits (5a, 5b, 5c) respectivement à un réservoir d'alimentation (P1) pour le sang à séparer et à au moins un réservoir de réception (P2, P3) du constituant séparé, des éléments de fermeture latérale étanche (6) de ce réseau de canaux (4a, 4b, 4c) et des moyens (7) pour faire circuler les liquides à travers ce réseau de canaux (4a, 4b, 4c), du réservoir d'alimentation (P1) aux réservoirs de réception (P2, P3), **caractérisé en ce que** les extrémités desdits conduits (5a, 5b, 5c) traversant ledit organe de mise en communication (5) de ladite enceinte (1) avec l'extérieur, concentrique audit axe de rotation, sont adjacentes aux extrémités respectives desdits canaux (4a, 4b, 4c), que les extrémités desdits conduits (5a, 5b, 5c) ont des surfaces complémentaires aux surfaces d'extrémités desdits canaux respectifs (4a, 4b, 4c) et que ces surfaces complémentaires sont fixées jointivement l'une à l'autre de manière étanche afin que ledit support (3) et ledit organe de mise en communication (5) de ladite enceinte (1) avec l'extérieur forment un ensemble rigide.

2. Ensemble selon la revendication 1, dans lequel les fixations entre les surfaces d'extrémités respectives desdits conduits (5a, 5b, 5c) et desdits canaux (4a, 4b, 4c) sont des fixations inamovibles.

3. Ensemble selon l'une des revendications précédentes, dans lequel ledit support (3) est venu d'une pièce avec un carter (2).

4. Ensemble selon la revendication 3, présentant une ouverture pour recevoir au moins partiellement l'enceinte de centrifugation, la partie de ce carter (2) opposée à celle qui est adjacente audit support (3) présentant une seconde ouverture pour permettre le passage dudit élément (1a) conformé pour venir en prise avec des moyens d'entraînement (11a) de la machine de centrifugation (M).

5. Ensemble selon l'une des revendications précédentes, dans lequel ledit support (3) est un support plan.

6. Ensemble selon les revendications 1 ou 2 et 5 dans lequel ledit support plan (3) est incliné par rapport à l'axe de rotation de ladite enceinte de centrifugation (1).

7. Ensemble selon les revendications 1 ou 2 et 5 dans lequel le support plan (3) est perpendiculaire à l'axe de rotation de ladite enceinte de centrifugation (1).
